(19) Europäisches Patentamt
European Patent Office
Office européen des brevets

(11) EP 3 326 999 B1

(12) # EUROPÄISCHE PATENTSCHRIFT

(45) Veröffentlichungstag und Bekanntmachung des Hinweises auf die Patenterteilung:
**07.08.2019 Patentblatt 2019/32**

(51) Int Cl.:
***C07C 229/22*** (2006.01) ***C07C 309/29*** (2006.01)
***C07C 309/30*** (2006.01)

(21) Anmeldenummer: **16200429.5**

(22) Anmeldetag: **24.11.2016**

(54) **SALICYLOYLCARNITIN SULFONATE**
SALICYLOYLCARNITIN SULFONATES
SALICYLOYLCARNITIN SULFONATES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.05.2018 Patentblatt 2018/22**

(73) Patentinhaber: **Drug'On Pharma Switzerland AG**
**4410 Liestal (CH)**

(72) Erfinder:
- **Meul, Thomas**
  **3930 Visp (CH)**
- **Weber, Peter**
  **4127 Birsfelden (CH)**

(74) Vertreter: **Latscha Schöllhorn Partner AG**
**Grellingerstrasse 60**
**4052 Basel (CH)**

(56) Entgegenhaltungen:
**EP-A1- 0 553 385**

EP 3 326 999 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

GEBIET DER ERFINDUNG

Die vorliegende Erfindung betrifft 3-(2-Hydroxybenzoyloxy)-4-(trimethylammonio)-buttersäurebetain-Hydrosulfonate der Formel (I) wie in der vorliegenden Beschreibung und den Ansprüchen definiert, sowie Verfahren zu deren Herstellung.

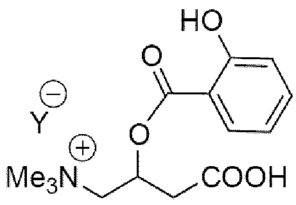

Formel (I)

HINTERGRUND DER ERFINDUNG

Salze des 3-(2-hydroxybenzoyloxy)-4-(trimethylammonio)-buttersäurebetains sind als Ester der Salicylsäure mit Carnitin (Salicyloylcarnitin) Salicylsäurederivate mit vielversprechenden therapeutischen Eigenschaften (siehe EP 553385, EP Anmeldung Nr. 16186393.1, eingereicht am 30.08.2016). Die Herstellung von Salicyloylcarnitin geht von L-Carnitin-HCl und dem Säurechlorid der Salicylsäure aus, deren Hydroxy-Gruppe durch eine Alkylgruppe geschützt ist. Je nachdem ob es sich bei dieser Alkyl-Schutzgruppe um eine primäre, sekundäre oder tertiäre Schutzgruppe handelt, gelingt deren Abspaltung mit gasförmigem Chlorwasserstoff oder mit hochkonzentrierter Bromwasserstoffsäure. Als Resultat dieser Abspaltung fällt das Hydrochlorid oder Hydrobromid des 3-(2-hydroxybenzoyloxy)-4-(trimethylammonio)-buttersäurebetains an.

Die so hergestellten Hydrohalogenide des 3-(2-hydroxybenzoyloxy)-4-(trimethylammonio)-buttersäurebetains enthalten wegen der bei der Herstellung als Lösungsmittel verwendeten halogenierten Essigsäuren einen Anteil an halogenierten Acetaten. Die Herstellung einheitlicher Salze oder des Betains selbst lassen sich durch eine wässrige Ionenaustauscher-Behandlung oder durch verlustreiche Umkristallisation erzielen.

Ein erheblicher Nachteil bei der Umkristallisation der Hydrohalogenide ist ihr Löslichkeitsverhalten. Beide Salze sind - wie Carnitin, bekannte Carnitin-Derivate und deren Salze - ausserordentlich gut wasserlöslich (>30% wt/wt), so dass eine Isolierung direkt aus wässrigen Lösungen nicht gelingt. Die Hydrohalogenide des 3-(2-hydroxybenzoyloxy)-4-(trimethylammonio)-buttersäurebetains werden daher aus wasserfreien organischen Medien gewonnen. Dabei muss man allerdings dem Umstand Rechnung tragen, dass potentielle Prozessverunreinigungen, wie das bei der Synthese als Ausgangsmaterial verwendete L-Carnitin, respektive dessen Salze oder die AlkylO-geschützten Zwischenprodukte, in organischen Medien schlechter löslich sind als die Hydrohalogenide des 3-(2-hydroxybenzoyloxy)-4-(trimethylammonio)-buttersäurebetains. Eine Abtrennung solcher Verunreinigungen durch Kristallisation der Hydrohalogenide des 3-(2-hydroxybenzoyloxy)-4-(trimethylammonio)-buttersäurebetains aus organischen Lösungsmitteln ist daher nur mit erheblichen Ausbeuteverlusten zu bewerkstelligen.

ZUSAMMENFASSUNG DER ERFINDUNG

Die vorliegende Erfindung stellt neue Arylsulfonate des 3-(2-hydroxybenzoyloxy)-4-(trimethylammonio)-buttersäurebetains bereit. Hierbei wurde nun überraschenderweise gefunden, dass die erfindungsgemässen Arylsulfonate des 3-(2-hydroxybenzoyloxy)-4-(trimethylammonio)-buttersäurebetains sich nicht nur überraschend als hydrophob erwiesen haben und in Wasser nur eine äusserst beschränkte Löslichkeit aufweisen, sondern dass diese auch in einfacher Weise durch Kristallisation aus Wasser erhalten werden können. Die erfindungsgemässen Hydrosulfonate stellen des Weiteren pharmakologisch tolerierbare Salze des 3-(2-Hydroxybenzoyloxy)-4-(trimethyl-ammonio)-buttersäurebetains dar und bieten sich daher in der Verwendung als Arzneimittel als direkte Alternativen zum bekannten 3-(2-hydroxybenzoyloxy)-4-(trimethylammonio)-buttersäurebetain-Hydrochlorid an.

Darüber hinaus ist ein weiterer Vorteil der erfindungsgemässen Arylsulfonate des 3-(2-hydroxybenzoyloxy)-4-(trimethylammonio)-buttersäurebetains und überraschend, dass sich entsprechende Arylsulfonate von potentiellen Prozessverunreinigungen wie die von L-Carnitin und AlkylO-geschützten Zwischenprodukten als so gut wasserlöslich herausstellten, dass diese im Gegensatz zu den erfindungsgemässen 3-(2-Hydroxybenzoyloxy)-4-(trimethylammonio)-buttersäurebetain-Hydrosulfonaten auch aus konzentrierten wässrigen Lösungen (>30% wt/wt) nicht auskristallisierten. Somit konnten besagte Prozessverunreinigungen bzw. deren Arylsulfonate durch Kristallisation der erfindungsgemässen Arylsulfonate aus Wasser in einfacher Weise und mit geringem Ausbeuteverlust eliminiert werden (siehe Beispiele 6 und 7).

Es wurde zudem überraschenderweise gefunden, dass Hydrohalogenide des 3-(2-Hydroxybenzoyloxy)-4-(trimethylammonio)-buttersäurebetains durch Umsetzung mit p-Toluolsulfonsäure oder Benzolsulfonsäure in Wasser, entweder nach vorheriger Neutralisation (also via Betain, siehe Beispiel 3) oder direkt (Beispiele 4, 5 und 8), in das entsprechende Hydrosulfonat überführt werden können.

In einem ersten Aspekt der Erfindung wird ein 3-(2-Hydroxybenzoyloxy)-4-(trimethylammonio)-buttersäurebetain-Hydrosulfonat der Formel (I) bereitgestellt,

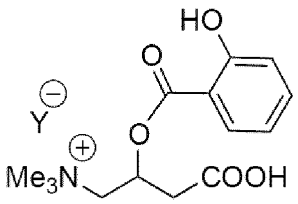

Formel (I)

wobei $Y^-$ ein Arylsulfonat $Ar\text{-}SO_3^-$ ist, wobei Ar für einen nicht-substituierten oder für einen C1-C4-alkyl-substituierten Phenylrest steht.

In einem zweiten Aspekt der Erfindung wird ein Verfahren zur Herstellung eines 3-(2-Hydroxybenzoyloxy)-4-(trimethylammonio)-buttersäurebetain-Hydrosulfonats der Formel (I) wie oben definiert bereitgestellt, umfassend Umsetzung eines 3-(2-Hydroxybenzoyloxy)-4-(trimethylammonio)-buttersäurebetain-Hydrohalogenids der Formel (II)

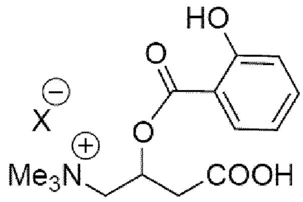

Formel (II)

wobei $X^-$ ausgewählt ist aus Chlorid ($Cl^-$), Bromid ($Br^-$) und Iodid ($I^-$), und wobei bevorzugt $X^-$ Chlorid ($Cl^-$) oder Bromid ($Br^-$) ist,
mit einer Arylsulfonsäure $Ar\text{-}SO_3H$, wobei Ar für einen nicht-substituierten oder für einen C1-C4-alkyl-substituierten Phenylrest steht,
in Wasser.

In einem dritten Aspekt der Erfindung wird ein Verfahren zur Herstellung eines 3-(2-Hydroxybenzoyloxy)-4-(trimethylammonio)-buttersäurebetain-Hydrosulfonats der Formel (I) wie oben definiert bereitgestellt, umfassend Umsetzung des 3-(2-Hydroxybenzoyloxy)-4-(trimethylammonio)-buttersäurebetains der Formel (III)

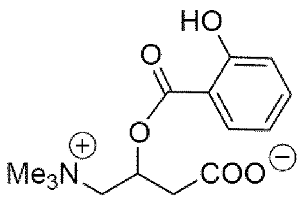

Formel (III)

mit einer Arylsulfonsäure $Ar\text{-}SO_3H$, wobei Ar für einen nicht-substituierten oder für einen C1-C4-alkyl-substituierten Phenylrest steht,
in Wasser.

In einem weiteren Aspekt der Erfindung wird ein 3-(2-Hydroxybenzoyloxy)-4-(trimethylammonio)-buttersäurebetain-Hydrosulfonat der Formel (I) wie oben definiert zur Verwendung als Arzneimittel bereitgestellt.

Weitere Aspekte und Ausführungsformen der Erfindung werden im Verlauf dieser Beschreibung offenkundig.

DETAILLIERTE BESCHREIBUNG DER ERFINDUNG

*Definitionen*

Der Begriff "Hydrosulfonat" bezieht sich auf Salze organischer Basen mit Arylsulfonsäuren Ar-$SO_3H$, wobei Ar für einen nicht-substituierten oder für einen C1-C4-alkyl-substituierten Phenylrest steht. Besonders bevorzugte Hydrosulfonate sind Hydrobesilate und Hydrotosylate.

Der Begriff "Arylsulfonat" bezieht sich auf ein Anion Ar-$SO_3^-$, wobei Ar für einen nicht-substituierten oder für einen C1-C4-alkyl-substituierten Phenylrest steht, bevorzugt für Phenyl oder für einen C1-C4-alkyl-substituierten Phenylrest ausgewählt aus *ortho*-Toluoyl, *meta*-Toluoyl, *para*-Toluoyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 4,5-Dimethylphenyl und Gemischen davon. Besonders bevorzugte Arylsulfonate Ar-$SO_3^-$ sind Tosylat (Ar ist *para*-Toluoyl) und Besilat (Ar ist Phenyl).

Der Begriff "Hydrobesilat" bezieht sich auf Salze organischer Basen mit Benzolsulfonsäure. "Besilat" ist entsprechend das Anion der Benzolsulfonsäure.

Der Begriff "Hydrotosylat" bezieht sich auf Salze organischer Basen mit *para*-Toluolsulfonsäure. "Tosylat" ist entsprechend das Anion der *para*-Toluolsulfonsäure.

Der Begriff "Raumtemperatur" bezieht sich auf eine Temperatur von 15 bis 30 °C, bevorzugt von 20 bis 25 °C.

*Salicyloylcarnitin Sulfonate*

Es wurde überraschenderweise gefunden, dass die erfindungsgemässen Arylsulfonate des 3-(2-hydroxybenzoyloxy)-4-(trimethylammonio)-buttersäurebetains sich nicht nur überraschend als hydrophob erwiesen haben und in Wasser nur eine äusserst beschränkte Löslichkeit aufweisen, sondern dass diese auch in einfacher Weise durch Kristallisation aus Wasser erhalten werden können. Im Gegensatz zu den erfindungsgemässen Arylsulfonaten des 3-(2-hydroxybenzoyloxy)-4-(trimethylammonio)-buttersäurebetains weisen die analogen Alkylsulfonate des 3-(2-hydroxybenzoyloxy)-4-(trimethylammonio)-buttersäurebetains, insbesondere das Mesylat und das Pentylsulfonat, eine sehr gute Wasserlöslichkeit auf (>30% w/w) und lassen sich darum nicht aus Wasser kristallisieren.

In einem ersten Aspekt der Erfindung wird ein 3-(2-Hydroxybenzoyloxy)-4-(trimethylammonio)-buttersäurebetain-Hydrosulfonat der Formel (I) bereitgestellt,

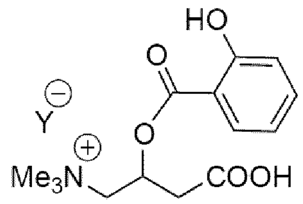

Formel (I)

wobei $Y^-$ ein Arylsulfonat Ar-$SO_3^-$ ist, wobei Ar für einen nicht-substituierten oder für einen C1-C4-alkyl-substituierten Phenylrest steht.

In einer Ausführungsform des ersten Aspekts der Erfindung wird ein 3-(2-Hydroxybenzoyloxy)-4-(trimethylammonio)-buttersäurebetain-Hydrosulfonat der Formel (I) bereitgestellt, wobei $Y^-$ ausgewählt ist aus Besilat, Tosylat, *ortho*-Toluolsulfonat, *meta*-Toluolsulfonat, 2,3-Dimethylbenzolsulfonat, 2,4-Dimethylbenzolsulfonat, 2,5-Dimethylbenzolsulfonat, 2,6-Dimethylbenzolsulfonat, 3,4-Dimethylbenzolsulfonat, 3,5-Dimethylbenzolsulfonat, 4,5-Dimethylbenzolsulfonat und Gemischen davon.

In einer bevorzugten Ausführungsform des ersten Aspekts der Erfindung wird ein 3-(2-Hydroxybenzoyloxy)-4-(trimethylammonio)-buttersäurebetain-Hydrosulfonat der Formel (I) bereitgestellt, wobei $Y^-$ ausgewählt ist aus Tosylat und Besilat.

In einer bevorzugten Ausführungsform des ersten Aspekts der Erfindung wird ein 3-(2-Hydroxybenzoyloxy)-4-(trimethylammonio)-buttersäurebetain-Hydrosulfonat der Formel (I) bereitgestellt, wobei $Y^-$ Besilat ist.

In einer bevorzugten Ausführungsform des ersten Aspekts der Erfindung wird ein 3-(2-Hydroxybenzoyloxy)-4-(trimethylammonio)-buttersäurebetain-Hydrosulfonat der Formel (I) bereitgestellt, wobei $Y^-$ Tosylat ist.

In einer besonders bevorzugten Ausführungsform des ersten Aspekts der Erfindung wird ein 3-(2-Hydroxybenzoyloxy)-4-(trimethylammonio)-buttersäurebetain-Hydrosulfonat der Formel (I) bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist; und

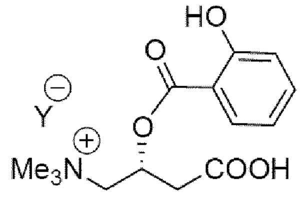

Formel (Ia)

wobei $Y^-$ ausgewählt ist aus Besilat und Tosylat.

In einer weiteren besonders bevorzugten Ausführungsform des ersten Aspekts der Erfindung wird ein 3-(2-Hydroxybenzoyloxy)-4-(trimethylammonio)-buttersäurebetain-Hydrosulfonat der Formel (I) bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist; und wobei $Y^-$ Besilat ist.

In einer weiteren besonders bevorzugten Ausführungsform des ersten Aspekts der Erfindung wird ein 3-(2-Hydroxybenzoyloxy)-4-(trimethylammonio)-buttersäurebetain-Hydrosulfonat der Formel (I) bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist; und wobei $Y^-$ Tosylat ist.

*Verfahren zur Herstellung von Salicyloylcarnitin Sulfonaten*

In einem zweiten Aspekt der Erfindung wird ein Verfahren zur Herstellung eines 3-(2-Hydroxybenzoyloxy)-4-(trimethylammonio)-buttersäurebetain-Hydrosulfonats der Formel (I) wie oben definiert bereitgestellt, umfassend Umsetzung eines 3-(2-Hydroxybenzoyloxy)-4-(trimethylammonio)-buttersäurebetain-Hydrohalogenids der Formel (II)

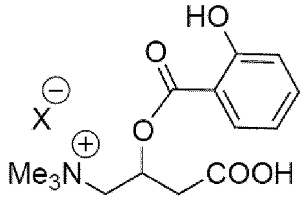

Formel (II)

wobei $X^-$ ausgewählt ist aus Chlorid ($Cl^-$), Bromid ($Br^-$) und Iodid ($I^-$), und wobei bevorzugt $X^-$ Chlorid ($Cl^-$) oder Bromid ($Br^-$) ist,

mit einer Arylsulfonsäure Ar-$SO_3H$, wobei Ar für einen nicht-substituierten oder für einen C1-C4-alkyl-substituierten Phenylrest steht,

in Wasser.

In einer Ausführungsform wird ein Verfahren nach dem zweiten Aspekt der Erfindung bereitgestellt, wobei das molare Verhältnis von 3-(2-Hydroxybenzoyloxy)-4-(trimethylammonio)-buttersäurebetain-Hydrohalogenid der Formel (II) zu besagter Arylsulfonsäure Ar-$SO_3H$ 1:1 bis 1: 2 beträgt.

In einem dritten Aspekt der Erfindung wird ein Verfahren zur Herstellung eines 3-(2-Hydroxybenzoyloxy)-4-(trimethylammonio)-buttersäurebetain-Hydrosulfonats der Formel (I) wie oben definiert bereitgestellt, umfassend Umsetzung des 3-(2-Hydroxybenzoyloxy)-4-(trimethylammonio)-buttersäurebetains der Formel (III)

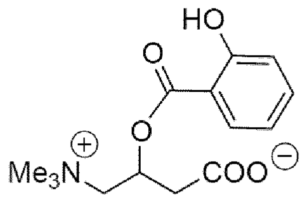

Formel (III)

mit einer Arylsulfonsäure Ar-$SO_3$H, wobei Ar für einen nicht-substituierten oder für einen C1-C4-alkyl-substituierten Phenylrest steht, in Wasser.

In einer Ausführungsform wird ein Verfahren nach dem dritten Aspekt der Erfindung bereitgestellt, wobei besagtes 3-(2-Hydroxybenzoyloxy)-4-(trimethylammonio)-buttersäurebetain der Formel (III) erhalten wird durch Umsetzung, typisch und bevorzugt durch Neutralisation (pH 7 bis 7.5 gemäss Beispiel 3), eines 3-(2-Hydroxybenzoyloxy)-4-(trimethylammonio)-buttersäurebetain-Hydrohalogenids der Formel (II);
wobei $X^-$ ausgewählt ist aus Chlorid ($Cl^-$), Bromid ($Br^-$) und Iodid ($I^-$); bevorzugt aus Chlorid ($Cl^-$) und Bromid ($Br^-$). mit einem Alkalimetallhydroxid, bevorzugt mit Natriumhydroxid (NaOH), wobei besagtes Alkalimetallhydroxid als Feststoff, z.B. in Pulverform, oder als wässrige Lösung, bevorzugt als wässrige Lösung, zugesetzt wird.

In einer bevorzugten Ausführungsform wird ein Verfahren nach dem dritten Aspekt der Erfindung bereitgestellt, wobei besagtes 3-(2-Hydroxybenzoyloxy)-4-(trimethylammonio)-buttersäurebetain der Formel (III) erhalten wird durch ein Verfahren umfassend

(a) Bereitstellen einer wässrigen Lösung eines 3-(2-Hydroxybenzoyloxy)-4-(trimethylammonio)-buttersäurebetain-Hydrohalogenids der Formel (II); wobei $X^-$ ausgewählt ist aus Chlorid ($Cl^-$), Bromid ($Br^-$) und Iodid ($I^-$);
(b) Einstellen des pH der Lösung auf 7-7.5 durch Zugabe einer verdünnten, typisch und bevorzugt einer 1-3 molaren, wässrigen Natriumhydroxid-Lösung.

In einer Ausführungsform wird ein Verfahren nach dem dritten Aspekt der Erfindung bereitgestellt, wobei das molare Verhältnis von 3-(2-Hydroxybenzoyloxy)-4-(trimethylammonio)-buttersäurebetain der Formel (III) zu besagter Arylsulfonsäure Ar-$SO_3$H 1:1 bis 1: 2 beträgt.

In einer Ausführungsform wird ein Verfahren nach dem zweiten oder dritten Aspekt der Erfindung bereitgestellt, wobei das molare Verhältnis von 3-(2-Hydroxybenzoyloxy)-4-(trimethylammonio)-buttersäurebetain-Hydrohalogenid der Formel (II) oder von 3-(2-Hydroxybenzoyloxy)-4-(trimethylammonio)-buttersäurebetain der Formel (III) zu besagter Arylsulfonsäure Ar-$SO_3$H 1:1 bis 1: 2 beträgt.

In einer Ausführungsform wird ein Verfahren nach dem zweiten oder dritten Aspekt der Erfindung bereitgestellt, weiter umfassend Isolierung des 3-(2-Hydroxybenzoyloxy)-4-(trimethylammonio)-buttersäurebetain-Hydrosulfonats der Formel (I) durch Kristallisation.

In einer Ausführungsform wird ein Verfahren nach dem zweiten oder dritten Aspekt der Erfindung bereitgestellt, weiter umfassend Isolierung des 3-(2-Hydroxybenzoyloxy)-4-(trimethylammonio)-buttersäurebetain-Hydrosulfonats der Formel (I) durch Kristallisation, wobei besagte Isolierung bei 0 bis 40°C, bevorzugt bei Raumtemperatur bis 40 °C, besonders bevorzugt bei Raumtemperatur durchgeführt wird.

In einer Ausführungsform wird ein Verfahren nach dem zweiten oder dritten Aspekt der Erfindung bereitgestellt, wobei besagte Umsetzung eines 3-(2-Hydroxybenzoyloxy)-4-(trimethylammonio)-buttersäurebetain-Hydrohalogenids der Formel (II) oder des 3-(2-Hydroxybenzoyloxy)-4-(trimethylammonio)-buttersäurebetains der Formel (III) mit einer Arylsulfonsäure Ar-$SO_3$H umfasst:

(a) Bereitstellen einer Lösung des besagten 3-(2-Hydroxybenzoyloxy)-4-(trimethylammonio)-buttersäurebetain-Hydrohalogenids der Formel (II) oder des 3-(2-Hydroxybenzoyloxy)-4-(trimethylammonio)-buttersäurebetains der Formel (III) in Wasser;
(b) Zugabe der besagten Arylsulfonsäure Ar-$SO_3$H, bevorzugt als Feststoff;
(c) Erhitzen des Reaktionsgemisches auf eine Temperatur oberhalb der Raumtemperatur, bevorzugt auf eine Temperatur von 70 bis 100 °C, besonders bevorzugt auf eine Temperatur von 70 bis 80 °C; und
(d) Isolierung des 3-(2-Hydroxybenzoyloxy)-4-(trimethylammonio)-buttersäurebetain-Hydrosulfonats der Formel (I) durch Kristallisation durch Abkühlen des Reaktionsgemisches mindestens auf Raumtemperatur, bevorzugt auf Raumtemperatur.

In einer bevorzugten Ausführungsform wird ein Verfahren nach dem zweiten Aspekt der Erfindung bereitgestellt,

wobei besagtes Hydrohalogenid der Formel (II) 3-(2-Hydroxybenzoyloxy)-4-(trimethylammonio)-buttersäurebetain-Hydrochlorid oder 3-(2-Hydroxybenzoyloxy)-4-(trimethylammonio)-buttersäurebetain-Hydrobromid ist.

In einer besonders bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrochlorid ist.

In einer weiteren besonders bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrobromid ist.

In einer bevorzugten Ausführungsform wird ein Verfahren nach dem zweiten oder dritten Aspekt der Erfindung bereitgestellt, wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrobesilat oder 3-(2-Hydroxybenzoyloxy)-4-(trimethylammonio)-buttersäurebetain-Hydrotosylat ist; und

wobei besagte Arylsulfonsäure Ar-$SO_3H$ Benzolsulfonsäure (Ar ist Phenyl) oder *para*-Toluolsulfonsäure (Ar ist *para*-Toluoyl) ist.

In einer besonders bevorzugten Ausführungsform wird ein Verfahren nach dem zweiten oder dritten Aspekt der Erfindung bereitgestellt, wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrobesilat ist und wobei besagte Arylsulfonsäure Ar-$SO_3H$ Benzolsulfonsäure (Ar ist Phenyl) ist.

In einer weiteren besonders bevorzugten Ausführungsform wird ein Verfahren nach dem zweiten oder dritten Aspekt der Erfindung bereitgestellt, wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrotosylat ist und wobei besagte Arylsulfonsäure Ar-$SO_3H$ *para*-Toluolsulfonsäure (Ar ist *para*-Toluoyl) ist.

In einer bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrochlorid oder 3-(2-Hydroxybenzoyloxy)-4-(trimethylammonio)-buttersäurebetain-Hydrobromid ist; und

wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrobesilat oder 3-(2-Hydroxybenzoyloxy)-4-(trimethylammonio)-buttersäurebetain-Hydrotosylat ist; und

wobei besagte Arylsulfonsäure Ar-$SO_3H$ Benzolsulfonsäure (Ar ist Phenyl) oder *para*-Toluolsulfonsäure (Ar ist *para*-Toluoyl) ist.

In einer weiteren bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrochlorid ist; und

wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrobesilat oder 3-(2-Hydroxybenzoyloxy)-4-(trimethylammonio)-buttersäurebetain-Hydrotosylat ist; und

wobei besagte Arylsulfonsäure Ar-$SO_3H$ Benzolsulfonsäure (Ar ist Phenyl) oder *para*-Toluolsulfonsäure (Ar ist *para*-Toluoyl) ist.

In einer weiteren bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrobromid ist; und

wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrobesilat oder 3-(2-Hydroxybenzoyloxy)-4-(trimethylammonio)-buttersäurebetain-Hydrotosylat ist; und

wobei besagte Arylsulfonsäure Ar-$SO_3H$ Benzolsulfonsäure (Ar ist Phenyl) oder *para*-Toluolsulfonsäure (Ar ist *para*-Toluoyl) ist.

In einer besonders bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrochlorid ist; und

wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrobesilat ist; und

wobei besagte Arylsulfonsäure Ar-$SO_3H$ Benzolsulfonsäure (Ar ist Phenyl) ist.

In einer weiteren besonders bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrochlorid ist; und

wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrotosylat ist; und

wobei besagte Arylsulfonsäure Ar-$SO_3H$ *para*-Toluolsulfonsäure (Ar ist *para*-Toluoyl) ist.

In einer weiteren besonders bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrobromid ist; und
wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrobesilat ist; und
wobei besagte Arylsulfonsäure Ar-$SO_3H$ Benzolsulfonsäure (Ar ist Phenyl) ist.

In einer weiteren besonders bevorzugten Ausführungsform wird ein Verfahren nach dem zweiten Aspekt der Erfindung bereitgestellt, wobei besagtes Hydrohalogenid der Formel (II) 3-(2-Hydroxybenzoyloxy)-4-(trimethylammonio)-buttersäurebetain-Hydrobromid ist; und
wobei besagtes 3-(2-Hydroxybenzoyloxy)-4-(trimethylammonio)-buttersäurebetain-Hydrosulfonat der Formel (I) 3-(2-Hydroxybenzoyloxy)-4-(trimethylammonio)-buttersäurebetain-Hydrotosylat ist; und
wobei besagte Arylsulfonsäure Ar-$SO_3H$ *para*-Toluolsulfonsäure (Ar ist *para*-Toluoyl) ist.

Das erfindungsgemässe Verfahren eignet sich selbstverständlich, je nach Ausgangsmaterial, gleichermassen für die Herstellung von racemischem wie von optisch aktivem 3-(2-hydroxybenzoyloxy)-4-(trimethylammonio)-buttersäurebetain-Hydrosulfonat. So eignet sich das erfindungsgemässe Verfahren nach dem zweiten oder dritten Aspekt der Erfindung besonders zur Herstellung eines (R)-(-)-3-(2-Hydroxybenzoyloxy)-4-trimethylammonio)-buttersäure-Hydrosulfonats der Formel (Ia), wobei $Y^-$ ein Arylsulfonat Ar-$SO_3^-$ ist, wobei Ar für einen nicht-substituierten oder für einen C1-C4-alkyl-substituierten Phenylrest steht.

Ganz besonders eignet sich das das erfindungsgemässe Verfahren nach dem zweiten oder dritten Aspekt der Erfindung zur Herstellung eines (R)-(-)-3-(2-Hydroxybenzoyloxy)-4-trimethylammonio)-buttersäure-Hydrosulfonats der Formel (Ia), wobei $Y^-$ ausgewählt ist aus Besilat und Tosylat.

Demzufolge wird in einer besonders bevorzugten Ausführungsform ein Verfahren nach dem zweiten Aspekt der Erfindung bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist, wobei $Y^-$ ausgewählt ist aus Besilat und Tosylat, und wobei besagte Arylsulfonsäure Ar-$SO_3H$ Benzolsulfonsäure (Ar = Phenyl) oder *para*-Toluolsulfonsäure (Ar = *para*-Toluoyl) ist und wobei besagtes 3-(2-Hydroxybenzoyloxy)-4-(trimethylammonio)-buttersäurebetain-Hydrohalogenid der Formel (II) ein (R)-(-)-3-(2-Hydroxybenzoyloxy)-4-(trimethylammonio)-buttersäurebetain-Hydrohalogenid der Formel (IIa) ist, wobei $X^-$ ausgewählt ist aus Chlorid ($Cl^-$), Bromid ($Br^-$) und Iodid ($I^-$), und wobei bevorzugt X Chlorid ($Cl^-$) oder Bromid ($Br^-$) ist.

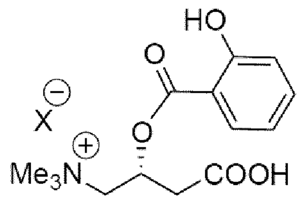

Formel (IIa)

In einer weiteren besonders bevorzugten Ausführungsform wird ein Verfahren nach dem zweiten Aspekt der Erfindung bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist, wobei $Y^-$ Besilat ist, und wobei besagte Arylsulfonsäure Ar-$SO_3H$ Benzolsulfonsäure (Ar = Phenyl) ist und wobei besagtes 3-(2-Hydroxybenzoyloxy)-4-(trimethylammonio)-buttersäurebetain-Hydrohalogenid der Formel (II) ein (R)-(-)-3-(2-Hydroxybenzoyloxy)-4-(trimethylammonio)-buttersäurebetain-Hydrohalogenid der Formel (IIa) ist, wobei $X^-$ ausgewählt ist aus Chlorid ($Cl^-$) und Bromid ($Br^-$).

In einer weiteren besonders bevorzugten Ausführungsform wird ein Verfahren nach dem zweiten Aspekt der Erfindung bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist, wobei $Y^-$ Tosylat ist, und wobei besagte Arylsulfonsäure Ar-$SO_3H$ *para*-Toluolsulfonsäure (Ar = *para*-Toluoyl) ist und wobei besagtes 3-(2-Hydroxybenzoyloxy)-4-(trimethylammonio)-buttersäurebetain-Hydrohalogenid der Formel (II) ein (R)-(-)-3-(2-Hydroxybenzoyloxy)-4-(trimethylammonio)-buttersäurebetain-Hydrohalogenid der Formel (IIa) ist, wobei $X^-$ ausgewählt ist aus Chlorid ($Cl^-$) und Bromid ($Br^-$).

In einer weiteren besonders bevorzugten Ausführungsform wird ein Verfahren nach dem dritten Aspekt der Erfindung bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist, wobei $Y^-$ ausgewählt ist aus Besilat und Tosylat, und wobei besagte Arylsulfonsäure Ar-$SO_3H$ Benzolsul-

fonsäure (Ar = Phenyl) oder *para*-Toluolsulfonsäure (Ar = *para*-Toluoyl) ist und wobei besagtes 3-(2-Hydroxybenzoyloxy)-4-(trimethylammonio)-buttersäurebetain der Formel (III) (R)-(-)-3-(2-Hydroxybenzoyloxy)-4-(trimethylammonio)-buttersäurebetain der Formel (IIIa) ist.

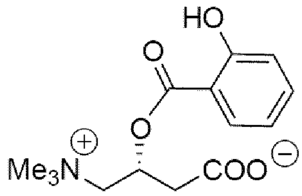

Formel (IIIa)

In einer weiteren besonders bevorzugten Ausführungsform wird ein Verfahren nach dem dritten Aspekt der Erfindung bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist, wobei $Y^-$ Besilat ist, und wobei besagte Arylsulfonsäure Ar-$SO_3H$ Benzolsulfonsäure (Ar = Phenyl) ist und wobei besagtes 3-(2-Hydroxybenzoyloxy)-4-(trimethylammonio)-buttersäurebetain der Formel (III) (R)-(-)-3-(2-Hydroxybenzoyloxy)-4-(trimethylammonio)-buttersäurebetain der Formel (IIIa) ist.

In einer weiteren besonders bevorzugten Ausführungsform wird ein Verfahren nach dem dritten Aspekt der Erfindung bereitgestellt, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist, wobei $Y^-$ Tosylat ist, und wobei besagte Arylsulfonsäure Ar-$SO_3H$ *para*-Toluolsulfonsäure (Ar = *para*-Toluoyl) ist und wobei besagtes 3-(2-Hydroxybenzoyloxy)-4-(trimethylammonio)-buttersäurebetain der Formel (III) (R)-(-)-3-(2-Hydroxybenzoyloxy)-4-(trimethylammonio)-buttersäurebetain der Formel (IIIa) ist.

*Verwendung der erfindungsgemässen Salicyloylcarnitin Sulfonate*

Die erfindungsgemässen Hydrosulfonate der Formel (I) sind pharmakologisch tolerierbare Salze des 3-(2-Hydroxybenzoyloxy)-4-(trimethyl-ammonio)-buttersäurebetains und bieten sich daher in der Verwendung als Arzneimittel als direkte Alternativen zum bekannten 3-(2-hydroxybenzoyloxy)-4-(trimethylammonio)-buttersäurebetain-Hydrochlorid an.

Folglich wird in einem weiteren Aspekt der Erfindung ein 3-(2-Hydroxybenzoyloxy)-4-(trimethylammonio)-buttersäurebetain-Hydrosulfonat der Formel (I) wie oben definiert zur Verwendung als Arzneimittel bereitgestellt.

BEISPIELE

Die Erfindung wird in den folgenden nicht-limitierenden Beispielen weiter erläutert.

BEISPIEL 1

**Reaktionsschemata**

Schema 1 zeigt das Verfahren zur Herstellung eines 3-(2-Hydroxybenzoyloxy)-4-(trimethylammonio)-buttersäurebetain-Hydrosulfonats der Formel (I) gemäss dem zweiten und dritten Aspekt der Erfindung, umfassend Umsetzung eines 3-(2-Hydroxybenzoyloxy)-4-(trimethylammonio)-buttersäurebetain-Hydrohalogenids der Formel (II), wobei $X^-$ ausgewählt ist aus Chlorid ($Cl^-$), Bromid ($Br^-$) und Iodid ($I^-$), wobei bevorzugt $X^-$ Chlorid ($Cl^-$) oder Bromid ($Br^-$) ist, oder des 3-(2-Hydroxybenzoyloxy)-4-(trimethylammonio)-buttersäurebetains der Formel (III) mit einer Arylsulfonsäure Ar-$SO_3H$, wobei Ar für einen nicht-substituierten oder für einen C1-C4-alkyl-substituierten Phenylrest steht, in Wasser.

## Schema 1

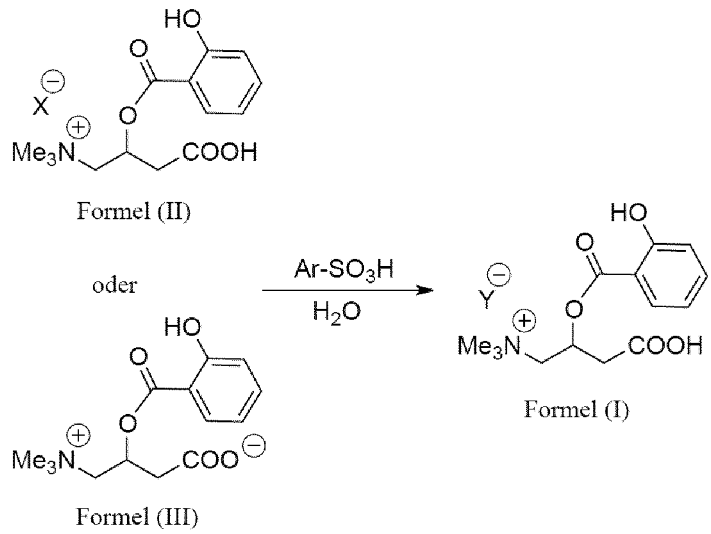


Formel (II)

oder

Formel (III)

Formel (I)

Schema 2 zeigt eine bevorzugte Ausführungsform des Verfahrens gemäss dem zweiten Aspekt der Erfindung. Zusätzlich gezeigt ist die Herstellung des Edukts für die bevorzugte Ausführungsform, nämlich (R)-(-)-3-(2-Hydroxybenzoyloxy)-4-(trimethylammonio)-buttersäurebetain-Hydrohalogenid der Formel (IIa), gemäss den in EP 553385 und der EP Anmeldung Nr. 16186393.1 (eingereicht am 30.08.2016) offenbarten Verfahren.

## Schema 2

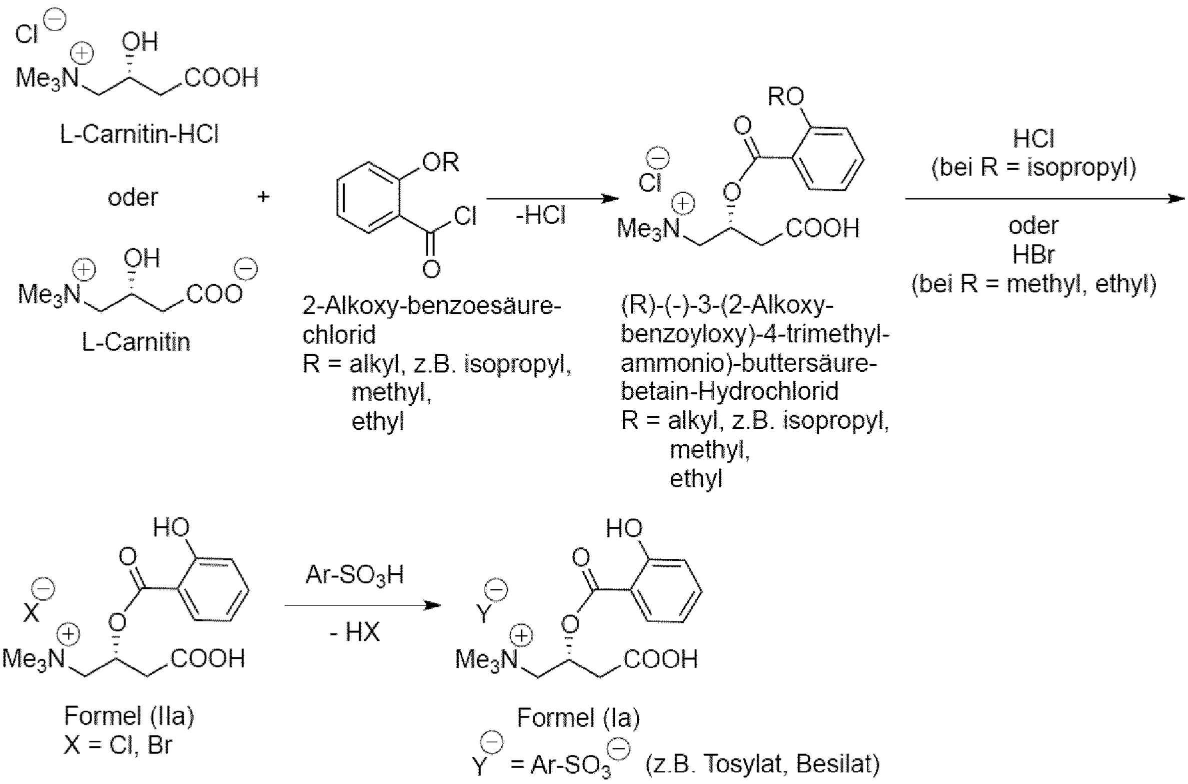


L-Carnitin-HCl

oder

+

L-Carnitin

2-Alkoxy-benzoesäure-chlorid
R = alkyl, z.B. isopropyl, methyl, ethyl

(R)-(-)-3-(2-Alkoxy-benzoyloxy)-4-trimethyl-ammonio)-buttersäure-betain-Hydrochlorid
R = alkyl, z.B. isopropyl, methyl, ethyl

Formel (IIa)
X = Cl, Br

Formel (Ia)
$Y^{\ominus}$ = Ar-$SO_3^{\ominus}$ (z.B. Tosylat, Besilat)

BEISPIEL 2

<u>**(R)-(-)-3-(2-Hydroxybenzoyloxy)-4-trimethylammonio)-buttersäurebetain-Hydrochlorid**</u>

In 50.0 g Dichloressigsäure wurden bei 60-65°C 9.9 g L-Carnitin-Hydrochlorid gelöst. Zu dieser Lösung wurden

bei -10 bis 0°C innerhalb von 60 Minuten 20.9 g 2-Isopropoxybenzoylchlorid zugetropft. Während des Zutropfens und der folgenden Nachreaktion bei -10 bis 0°C wurde durch Anlegen eines schwachen Vakuums (100-200 mbar) das freiwerdende Chlorwasserstoff-Gas aus der Reaktionsmischung vertrieben. Die Reaktion wurde mittels DC verfolgt (Kieselgel; Laufmittel: Chloroform:Methanol: Wasser:5%ige Ammoniaklösung 55:39:1:5). Nach ca. 1 Stunde wurden 9.4 g Salzsäure-Gas in die Reaktionslösung eingeleitet. Die Reaktionslösung wurde 30 Minuten auf 45-50°C erhitzt, mit geringen Mengen an Wasser versetzt und bei 20 bis 25°C weiter gerührt. Die Reaktion wurde wiederum mittels DC verfolgt (Kieselgel; Laufmittel: Chloroform:Methanol:Wasser:5%ige Ammoniaklösung 55:39:1:5). Anschliessend wurde die Reaktionsmischung mit 200 ml Essigsäureethylester verdünnt und mit Salicyloyl-L-Carnitin-HCl angeimpft. Sobald das Produkt anfing zu kristallisieren, wurden weitere 200 ml Essigsäureethylester hinzugefügt. Die entstandene Suspension wurde auf 10 bis 15°C abgekühlt und noch eine Stunde bei dieser Temperatur gerührt. Das auskristallisierte Produkt wurde abgenutscht, mit 100 ml Essigsäureethylester gewaschen und im Vakuumtrockenschrank bei 40-45°C über Nacht getrocknet.
Ausbeute: 14.6 g (92%)
Smp. : 184-185°C $R_F$: 0.19 (Kieselgel; Laufmittel: Chloroform:Methanol:Wasser: 5%ige $NH_3$ lösung 55:39:1:5)

$[\alpha]_D^{20}$ = -25.6° (c=1, Wasser) pH (1% in $H_2O$): 2.5

BEISPIEL 3

**(R)-(-)-3-(2-Hydroxybenzoyloxy)-4-trimethylammonio)-buttersäurebetain-Hydrotosylat**

1120 mg (R)-(-)-3-(2-Hydroxybenzoyloxy)-4-trimethylammonio)-buttersäurebetain-Hydrochlorid aus Beispiel 2 werden in 4.4 mL Wasser gelöst und der pH der Lösung mit verdünnter NaOH, typisch 1-3 molarer NaOH, auf 7 bis 7.5 gestellt. Zu dieser Lösung gibt man 670 mg p-Toluolsulfonsäure-Monohydrat. Die entstandene Suspension wird auf 70-80 °C erhitzt, wobei vollständige Löslichkeit eintritt. Beim Abkühlen auf Raumtemperatur kristallisiert das Hydrotosylat des (R)-(-)-3-(2-Hydroxybenzoyloxy)-4-trimethylammonio)-buttersäurebetains als weisser Feststoff aus. Das ausgefallene Produkt wurde abgenutscht und im Vakuum bei 40-45°C getrocknet.
Ausbeute: 1430 mg (90%)

Smp.: 197-199°C $[\alpha]_D^{20}$ = -20.9° (c=1, Wasser) $^1$H-NMR (DMSO-$d_6$, 300 MHz) $\delta$ : 7.83 (d, 1H); 7.56 (t, 1H); 7.48 (d, 2H); 7.12 (d, 2H); 7.02 (d, 1H); 6.97 (t, 1H); 5.76-5.67 (m, 1H); 4.10-3.70 (m, 2H); 3.16 (s, 9H); 2.96-2.78 (m, 2H); 2.29 (s, 3H).
pH (1% in $H_2O$): 2.6
Löslichkeit (bei 30°C): 2.3g in 100 mL $H_2O$

BEISPIEL 4

**(R)-(-)-3-(2-Hydroxybenzoyloxy)-4-trimethylammonio)-buttersäurebetain-Hydrotosylat**

560 mg (R)-(-)-3-(2-Hydroxybenzoyloxy)-4-trimethylammonio)-buttersäurebetain-Hydrochlorid aus Beispiel 2 werden in 3 mL Wasser gelöst. Zu dieser Lösung gibt man 380 mg p-Toluolsulfonsäure-Monohydrat. Die entstandene Suspension wird auf 70-80 °C erhitzt, wobei vollständige Löslichkeit eintritt. Beim Abkühlen auf Raumtemperatur kristallisiert das Hydrotosylat des (R)-(-)-3-(2-Hydroxybenzoyloxy)-4-trimethylammonio)-buttersäurebetains als weisser Feststoff aus. Das ausgefallene Produkt wurde abgenutscht und im Vakuum bei 40-45°C getrocknet.
Ausbeute: 800 mg (88%)
Smp.: 197-199°C

BEISPIEL 5

**(R)-(-)-3-(2-Hydroxybenzoyloxy)-4-trimethylammonio)-buttersäurebetain-Hydrobesilat**

1270 mg (R)-(-)-3-(2-Hydroxybenzoyloxy)-4-trimethylammonio)-buttersäurebetain-Hydrochlorid aus Beispiel 2 und 670 mg Benzolsulfonsäure werden in 8 mL Wasser vorgelegt. Die entstandene Suspension wird auf 70-80 °C erhitzt, wobei vollständige Löslichkeit eintritt. Beim Abkühlen auf Raumtemperatur kristallisiert das Hydrobesilat des (R)-(-)-3-(2-Hydroxybenzoyloxy)-4-trimethylammonio)-buttersäurebetains als weisser Feststoff aus. Das ausgefallene Produkt wurde abgenutscht und im Vakuum bei 40-45°C getrocknet.

Ausbeute: 1.4 g (80%)

Smp.: 131-132°C $[\alpha]_D^{20}$ = -21.8° (c=1, Wasser) $^1$H-NMR ($D_2O$, 300 MHz) δ : 7.85-6.91 (m, 9H); 5.93-5.84 (m, 1H); 4.10-3.68 (m, 2H); 3.16 (s, 9H); 2.99-2.80 (m, 2H).
pH (1% in $H_2O$): 2.6
Löslichkeit (bei 30°C): 6.1g in 100 mL $H_2O$

BEISPIEL 6

**(R)-(-)-3-(2-Hydroxybenzoyloxy)-4-trimethylammonio)-buttersäurebetain-Hydrotosylat**

640 mg Salicyloyl-L-carnitin-Hydrochlorid aus Beispiel 2, 340 mg L-Carnitin und 870 mg p-Toluolsulfonsäure-Monohydrat werden in 5.2 mL Wasser heiss gelöst. Beim Abkühlen auf Raumtemperatur kristallisiert das Hydrotosylat des (R)-(-)-3-(2-Hydroxybenzoyloxy)-4-trimethylammonio)-buttersäurebetains als weisser Feststoff aus. Das ausgefallene Produkt wurde abgenutscht, mit wenig Wasser gewaschen und im Vakuum bei 40-45°C getrocknet.
Ausbeute: 810 mg (89%)
Smp.: 197-199°C

BEISPIEL 7

**(R)-(-)-3-(2-Hydroxybenzoyloxy)-4-trimethylammonio)-buttersäurebetain-Hydrotosylat**

640 mg Salicyloyl-L-carnitin-Hydrochlorid aus Beispiel 2, 370 mg (R)-(-)-3-(2-Isopropoxybenzoyloxy)-4-trimethylammonio)-buttersäurebetain und 610 mg p-Toluolsulfonsäure-Monohydrat werden in 4.5 mL Wasser heiss gelöst. Beim Abkühlen auf Raumtemperatur kristallisierte das Hydrotosylat des (R)-(-)-3-(2-Hydroxybenzoyloxy)-4-trimethylammonio)-buttersäurebetains als weisser Feststoff aus. Das ausgefallene Produkt wurde abgenutscht, mit wenig Wasser gewaschen und im Vakuum bei 40-45°C getrocknet.
Ausbeute: 650 mg (71%)
Smp.: 197-199°C

BEISPIEL 8

**(R)-(-)-3-(2-Hydroxybenzoyloxy)-4-trimethylammonio)-buttersäurebetain-Hydrotosylat**

363 mg (R)-(-)-3-(2-Hydroxybenzoyloxy)-4-trimethylammonio)-buttersäurebetain-Hydrobromid, hergestellt nach dem in EP 553385 beschriebenen Verfahren, werden in 1.5 mL Wasser gelöst. Zu dieser Lösung gibt man 190 mg p-Toluolsulfonsäure-Monohydrat. Die entstandene Suspension wird erhitzt, wobei vollständige Löslichkeit eintritt. Beim Abkühlen auf Raumtemperatur kristallisiert das Hydrotosylat des (R)-(-)-3-(2-Hydroxybenzoyloxy)-4-trimethylammonio)-buttersäurebetains als weisser Feststoff aus. Das ausgefallene Produkt wurde abgenutscht, mit wenig Wasser gewaschen und im Vakuum bei 40-45°C getrocknet.
Ausbeute: 320 mg (88%)
Smp.: 197-199°C

## Patentansprüche

**1.** 3-(2-Hydroxybenzoyloxy)-4-(trimethylammonio)-buttersäurebetain-Hydrosulfonat der Formel (I)

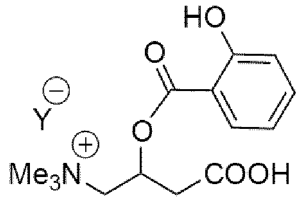

Formel (I)

wobei $Y^-$ ein Arylsulfonat $Ar\text{-}SO_3^-$ ist, wobei Ar für einen nicht-substituierten oder für einen C1-C4-alkyl-substituierten Phenylrest steht.

2. 3-(2-Hydroxybenzoyloxy)-4-(trimethylammonio)-buttersäurebetain-Hydrosulfonat der Formel (I) nach Anspruch 1, wobei $Y^-$ ausgewählt ist aus Tosylat und Besilat.

3. 3-(2-Hydroxybenzoyloxy)-4-(trimethylammonio)-buttersäurebetain-Hydrosulfonat der Formel (I) nach Anspruch 1, wobei $Y^-$ Besilat ist.

4. 3-(2-Hydroxybenzoyloxy)-4-(trimethylammonio)-buttersäurebetain-Hydrosulfonat der Formel (I) nach Anspruch 1, wobei Y Tosylat ist.

5. 3-(2-Hydroxybenzoyloxy)-4-(trimethylammonio)-buttersäurebetain-Hydrosulfonat der Formel (I) nach Anspruch 1, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist; und

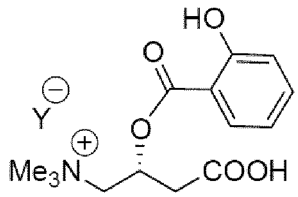

Formel (Ia)

wobei $Y^-$ ausgewählt ist aus Besilat und Tosylat.

6. Verfahren zur Herstellung eines 3-(2-Hydroxybenzoyloxy)-4-(trimethylammonio)-buttersäurebetain-Hydrosulfonats der Formel (I) nach Anspruch 1, umfassend Umsetzung eines 3-(2-Hydroxybenzoyloxy)-4-(trimethylammonio)-buttersäurebetain-Hydrohalogenids der Formel (II)

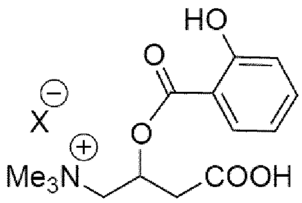

Formel (II)

wobei $X^-$ ausgewählt ist aus Chlorid ($Cl^-$), Bromid ($Br^-$) und Iodid ($I^-$), wobei bevorzugt $X^-$ Chlorid ($Cl^-$) oder Bromid ($Br^-$) ist,
mit einer Arylsulfonsäure $Ar\text{-}SO_3H$, wobei Ar für einen nicht-substituierten oder für einen C1-C4-alkyl-substituierten Phenylrest steht, in Wasser.

7. Verfahren zur Herstellung eines 3-(2-Hydroxybenzoyloxy)-4-(trimethylammonio)-buttersäurebetain-Hydrosulfonats

der Formel (I) nach Anspruch 1, umfassend Umsetzung des 3-(2-Hydroxybenzoyloxy)-4-(trimethylammonio)-buttersäurebetains der Formel (III)

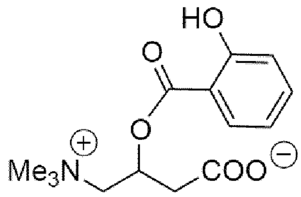

Formel (III)

mit einer Arylsulfonsäure Ar-$SO_3$H, wobei Ar für einen nicht-substituierten oder für einen C1-C4-alkyl-substituierten Phenylrest steht, in Wasser.

**8.** Verfahren nach Anspruch 7, wobei besagtes 3-(2-Hydroxybenzoyloxy)-4-(trimethylammonio)-buttersäurebetain der Formel (III) erhalten wird durch Umsetzung eines 3-(2-Hydroxybenzoyloxy)-4-(trimethylammonio)-buttersäurebetain-Hydrohalogenids der Formel (II) mit einem Alkalimetallhydroxid, wobei $X^-$ ausgewählt ist aus Chlorid ($Cl^-$), Bromid ($Br^-$) und Iodid ($I^-$), und wobei bevorzugt besagtes Alkalimetallhydroxid Natriumhydroxid (NaOH) ist.

**9.** Verfahren nach einem der Ansprüche 6 bis 8, wobei das molare Verhältnis von 3-(2-Hydroxybenzoyloxy)-4-(trimethylammonio)-buttersäurebetain-Hydrohalogenid der Formel (II) oder von 3-(2-Hydroxybenzoyloxy)-4-(trimethylammonio)-buttersäurebetain der Formel (III) zu besagter Arylsulfonsäure Ar-$SO_3$H 1:1 bis 1: 2 beträgt.

**10.** Verfahren nach einem der Ansprüche 6 bis 9, weiter umfassend Isolierung des 3-(2-Hydroxybenzoyloxy)-4-(trimethylammonio)-buttersäurebetain-Hydrosulfonats der Formel (I) durch Kristallisation.

**11.** Verfahren nach Anspruch 10, wobei besagte Isolierung bei 0 bis 40°C durchgeführt wird.

**12.** Verfahren nach einem der Ansprüche 6 bis 11, wobei besagte Umsetzung eines 3-(2-Hydroxybenzoyloxy)-4-(trimethylammonio)-buttersäurebetain-Hydrohalogenids der Formel (II) oder des 3-(2-Hydroxybenzoyloxy)-4-(trimethylammonio)-buttersäurebetains der Formel (III) mit einer Arylsulfonsäure Ar-$SO_3$H umfasst:

(a) Bereitstellen einer Lösung des besagten 3-(2-Hydroxybenzoyloxy)-4-(trimethylammonio)-buttersäurebetain-Hydrohalogenids der Formel (II) oder des 3-(2-Hydroxybenzoyloxy)-4-(trimethylammonio)-buttersäurebetains der Formel (III) in Wasser;
(b) Zugabe der besagten Arylsulfonsäure Ar-$SO_3$H;
(c) Erhitzen des Reaktionsgemisches auf eine Temperatur oberhalb der Raumtemperatur; und
(d) Isolierung des 3-(2-Hydroxybenzoyloxy)-4-(trimethylammonio)-buttersäurebetain-Hydrosulfonats der Formel (I) durch Kristallisation durch Abkühlen des Reaktionsgemisches mindestens auf Raumtemperatur,

wobei der Begriff Raumtemperatur sich auf eine Temperatur von 15 bis 30°C bezieht.

**13.** Verfahren nach einem der Ansprüche 6 und 8 bis 12, wobei besagtes Hydrohalogenid der Formel (II) 3-(2-Hydroxybenzoyloxy)-4-(trimethylammonio)-buttersäurebetain-Hydrochlorid ist.

**14.** Verfahren nach einem der Ansprüche 6 und 8 bis 13, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrobesilat der Formel (Ia) ist, wobei Y ausgewählt ist aus Besilat und Tosylat und wobei besagte Arylsulfonsäure Ar-$SO_3$H Benzolsulfonsäure oder *para*-Toluolsulfonsäure ist und wobei besagtes 3-(2-Hydroxybenzoyloxy)-4-(trimethylammonio)-buttersäurebetain-Hydrohalogenid der Formel (II) ein (R)-(-)-3-(2-Hydroxybenzoyloxy)-4-(trimethylammonio)-buttersäurebetain-Hydrohalogenid der Formel (IIa) ist, wobei $X^-$ ausgewählt ist aus Chlorid ($Cl^-$), Bromid ($Br^-$) und Iodid ($I^-$), und wobei bevorzugt X Chlorid ($Cl^-$) oder Bromid ($Br^-$) ist.

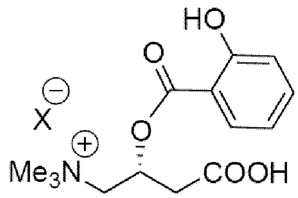

Formel (IIa)

**15.** Verfahren nach einem der Ansprüche 7 bis 13, wobei besagtes 3-(2-Hydroxybenzoyloxy)-4-(trimethylammonio)-buttersäurebetain-Hydrosulfonat der Formel (I) ein (R)-(-)-3-(2-Hydroxybenzoyloxy)-4-(trimethylammonio)-buttersäurebetain-Hydrosulfonat der Formel (Ia) ist, wobei $Y^-$ ausgewählt ist aus Besilat und Tosylat und wobei besagte Arylsulfonsäure Ar-$SO_3$H Benzolsulfonsäure oder *para*-Toluolsulfonsäure ist und wobei besagtes 3-(2-Hydroxybenzoyloxy)-4-(trimethylammonio)-buttersäurebetain der Formel (III) (R)-(-)-3-(2-Hydroxybenzoyloxy)-4-(trimethylammonio)-buttersäurebetain der Formel (IIIa) ist.

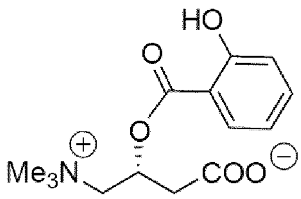

Formel (IIIa)

## Claims

**1.** A 3-(2-hydroxybenzoyloxy)-4-(trimethylammonio)-butyric acid betaine hydrosulfonate of formula (I)

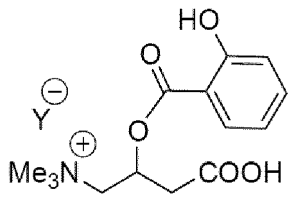

Formula (I)

where $Y^-$ is an arylsulfonate Ar-$SO_3^-$, where Ar stands for an unsubstituted phenyl functional group or for a C1-C4 alkyl-substituted phenyl functional group.

**2.** The 3-(2-hydroxybenzoyloxy)-4-(trimethylammonio)-butyric acid betaine hydrosulfonate of formula (I) according to Claim 1, where $Y^-$ is selected from tosylate and besylate.

**3.** The 3-(2-hydroxybenzoyloxy)-4-(trimethylammonio)-butyric acid betaine hydrosulfonate of formula (I) according to Claim 1, where $Y^-$ is besylate.

**4.** The 3-(2-hydroxybenzoyloxy)-4-(trimethylammonio)-butyric acid betaine hydrosulfonate of formula (I) according to Claim 1, where $Y^-$ is tosylate.

**5.** The 3-(2-hydroxybenzoyloxy)-4-(trimethylammonio)-butyric acid betaine hydrosulfonate of formula (I) according to Claim 1, wherein the stated 3-(2-hydroxybenzoyloxy)-4-(trimethylammonio)-butyric acid betaine hydrosulfonate of formula (I) is an (*R*)-(-)-3-(2-hydroxybenzoyloxy)-4-(trimethylammonio)-butyric acid betaine hydrosulfonate of formula (Ia);

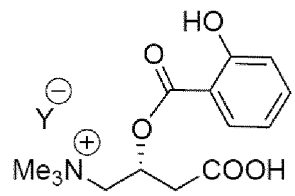

Formula (Ia)

where $Y^-$ is selected from besylate and tosylate.

6. A method for preparing a 3-(2-hydroxybenzoyloxy)-4-(trimethylammonio)-butyric acid betaine hydrosulfonate of formula (I) according to Claim 1, comprising reacting a 3-(2-hydroxybenzoyloxy)-4-(trimethylammonio)-butyric acid betaine hydrohalide of formula (II)

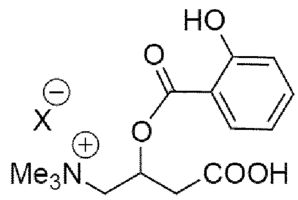

Formula (II)

where $X^-$ is selected from chloride ($Cl^-$), bromide ($Br^-$), and iodide ($I^-$), with $X^-$ preferably being chloride ($Cl^-$) or bromide ($Br^-$),
with an arylsulfonic acid Ar-$SO_3H$, where Ar stands for an unsubstituted phenyl functional group or for a C1-C4 alkylsubstituted phenyl functional group, in water.

7. A method for preparing a 3-(2-hydroxybenzoyloxy)-4-(trimethylammonio)-butyric acid betaine hydrosulfonate of formula (I) according to Claim 1, comprising reacting the 3-(2-hydroxybenzoyloxy)-4-(trimethylammonio)-butyric acid betaine of formula (III)

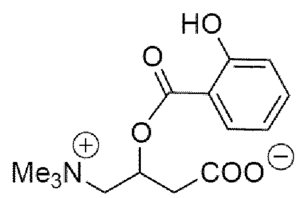

Formula (III)

with an arylsulfonic acid Ar-$SO_3H$, where Ar stands for an unsubstituted phenyl functional group or for a C1-C4 alkylsubstituted phenyl functional group, in water.

8. The method according to Claim 7, wherein the stated 3-(2-hydroxybenzoyloxy)-4-(trimethylammonio)-butyric acid betaine of formula (III) is obtained by reacting a 3-(2-hydroxybenzoyloxy)-4-(trimethylammonio)-butyric acid betaine hydrohalide of formula (II) with an alkali metal hydroxide, where $X^-$ is selected from chloride ($Cl^-$), bromide ($Br^-$), and iodide ($I^-$), and where the stated alkali metal hydroxide is preferably sodium hydroxide (NaOH).

9. The method according to any one of Claims 6 to 8, wherein the molar ratio of 3-(2-hydroxybenzoyloxy)-4-(trimethylammonio)-butyric acid betaine hydrohalide of formula (II) or of 3-(2-hydroxybenzoyloxy)-4-(trimethylammonio)-butyric acid betaine of formula (III) to the stated arylsulfonic acid Ar-$SO_3H$ is 1:1 to 1: 2.

10. The method according to any one of Claims 6 to 9, further comprising isolation of the 3-(2-hydroxybenzoyloxy)-4-(trimethylammonio)-butyric acid betaine hydrosulfonate of formula (I) by crystallization.

**11.** The method according to Claim 10, wherein the stated isolation is carried out at 0 to 40°C.

**12.** The method according to any one of Claims 6 to 11, wherein the stated reaction of a 3-(2-hydroxybenzoyloxy)-4-(trimethylammonio)-butyric acid betaine hydrohalide of formula (II) or of the 3-(2-hydroxybenzoyloxy)-4-(trimethylammonio)-butyric acid betaine of formula (III) with an arylsulfonic acid Ar-$SO_3$H comprises:

(a) Providing a solution of the stated 3-(2-hydroxybenzoyloxy)-4-(trimethylammonio)-butyric acid betaine hydrohalide of formula (II) or of the 3-(2-hydroxybenzoyloxy)-4-(trimethylammonio)-butyric acid betaine of formula (III) in water;
(b) Adding the stated arylsulfonic acid Ar-$SO_3$H;
(c) Heating the reaction mixture to a temperature above room temperature; and
(d) Isolating the 3-(2-hydroxybenzoyloxy)-4-(trimethylammonio)-butyric acid betaine hydrosulfonate of formula (I) by crystallization by cooling the reaction mixture at least to room temperature,

wherein the term "room temperature" refers to a temperature of 15 to 30°C.

**13.** The method according to any one of Claims 6 and 8 to 12, wherein the stated hydrohalide of formula (II) is 3-(2-hydroxybenzoyloxy)-4-(trimethylammonio)-butyric acid betaine hydrochloride.

**14.** The method according to any one of Claims 6 and 8 to 13, wherein the stated 3-(2-hydroxybenzoyloxy)-4-(trimethylammonio)-butyric acid betaine hydrosulfonate of formula (I) is an (*R*)-(-)-3-(2-hydroxybenzoyloxy)-4-(trimethylammonio)-butyric acid betaine hydrobesylate of formula (Ia), where $Y^-$ is selected from besylate and tosylate, and where the stated arylsulfonic acid Ar-$SO_3$H is benzenesulfonic acid or para-toluenesulfonic acid, and where the stated 3-(2-hydroxybenzoyloxy)-4-(trimethylammonio)-butyric acid betaine hydrohalide of formula (II) is an (*R*)-(-)-3-(2-hydroxybenzoyloxy)-4-(trimethylammonio)-butyric acid betaine hydrohalide of formula (IIa), where $X^-$ is selected from chloride ($Cl^-$), bromide ($Br^-$), and iodide ($I^-$), and where $X^-$ is preferably chloride ($Cl^-$) or bromide ($Br^-$).

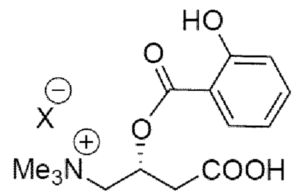

Formula (IIa)

**15.** The method according to any one of Claims 7 to 13, wherein the stated 3-(2-hydroxybenzoyloxy)-4-(trimethylammonio)-butyric acid betaine hydrosulfonate of formula (I) is an (*R*)-(-)-3-(2-hydroxybenzoyloxy)-4-(trimethylammonio)-butyric acid betaine hydrosulfonate of formula (Ia), where $Y^-$ is selected from besylate and tosylate, and where the stated arylsulfonic acid Ar-$SO_3$H is benzenesulfonic acid or para-toluenesulfonic acid, and where the stated 3-(2-hydroxybenzoyloxy)-4-(trimethylammonio)-butyric acid betaine of formula (III) is (R)-(-)-3-(2-hydroxybenzoyloxy)-4-(trimethylammonio)-butyric acid betaine of formula (IIIa).

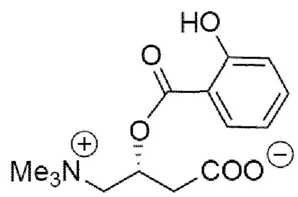

Formula (IIIa)

## Revendications

1. Hydrosulfonate de bétaïne d'acide béta-3-(2-hydroxybenzoyloxy)-4-(triméthylammonio)-butyrique de formule (I)

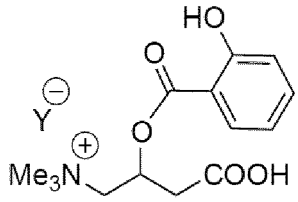

Formule (I)

$Y^-$ étant un sulfonate d'aryle $Ar\text{-}SO_3^-$, Ar étant un radical phényle non substitué ou substitué par un alkyle en $C_1$ à $C_4$.

2. Hydrosulfonate de bétaïne d'acide 3-(2-hydroxybenzoyloxy)-4-(triméthylammonio)-butyrique de formule (I) selon la revendication 1, $Y^-$ étant choisi parmi le tosylate et le bésilate.

3. Hydrosulfonate de bétaïne d'acide 3-(2-hydroxybenzoyloxy)-4-(triméthylammonio)-butyrique de formule (I) selon la revendication 1, $Y^-$ étant un bésylate.

4. Hydrosulfonate de bétaïne d'acide 3-(2-hydroxybenzoyloxy)-4-(triméthylammonio)-butyrique de formule (I) selon la revendication 1, $Y^-$ étant un tosylate.

5. Hydrosulfonate de bétaïne d'acide 3-(2-hydroxybenzoyloxy)-4-(triméthylammonio)-butyrique de formule (I) selon la revendication 1, ledit hydrosulfonate de bétaïne d'acide 3-(2-hydroxybenzoyloxy)-4-(triméthylammonio)-butyrique de formule (I) étant un hydrosulfonate de bétaïne d'acide (R)-(-)-3-(2-hydroxybenzoyloxy)-4-(triméthylammonio)-butyrique de formule (Ia) ; et

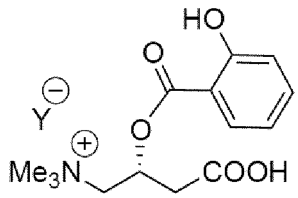

Formule (Ia)

$Y^-$ étant choisi parmi le besilate et le tosylate.

6. Procédé de fabrication d'un hydrosulfonate de bétaïne d'acide 3-(2-hydroxybenzoyloxy)-4-(triméthylammonio)-butyrique de formule (I) selon la revendication 1, comprenant la réaction d'un hydrohalogénure de bétaïne d'acide 3-(2-hydroxybenzoyloxy)-4-(triméthylammonio)-butyrique de formule (II)

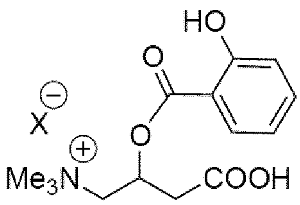

Formule (II)

$X^-$ étant choisi parmi le chlorure ($Cl^-$), le bromure ($Br^-$) et l'iodure ($I^-$), de préférence $X^-$ étant le chlorure ($Cl^-$) ou le bromure ($Br^-$),

avec un acide arylsulfonique Ar-$SO_3H$, Ar étant un radical phényle non substitué ou substitué par un alkyle en $C_1$ à C4, dans de l'eau.

**7.** Procédé de préparation d'un hydrosulfonate de bétaïne d'acide 3-(2-hydroxybenzoyloxy)-4-(triméthylammonio)-butyrique de formule (I) selon la revendication 1, comprenant la réaction de la bétaïne d'acide 3-(2-hydroxybenzoyloxy)-4-(triméthylammonio)-butyrique de la formule (III)

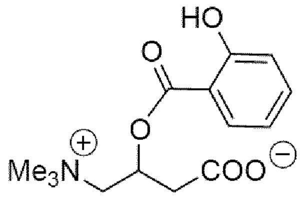

Formule (III)

avec un acide arylsulfonique Ar-$SO_3H$, Ar étant un radical phényle non substitué ou substitué par un alkyle en $C_1$ à $C_4$, dans de l'eau.

**8.** Procédé selon la revendication 7, ladite bétaïne d'acide 3-(2-hydroxybenzoyloxy)-4-(triméthylammonio)-butyrique de formule (III) étant obtenue par réaction d'un hydrohalogénure de bétaïne d'acide 3-(2-hydroxybenzoyloxy)-4-(triméthylammonio)-butyrique de formule (II) avec un hydroxyde de métal alcalin, $X^-$ étant choisi parmi le chlorure ($Cl^-$), le bromure ($Br^-$) et l'iodure ($I^-$), et de préférence ledit hydroxyde de métal alcalin étant l'hydroxyde de sodium (NaOH).

**9.** Procédé selon l'une des revendications 6 à 8, le rapport molaire de l'hydrohalogénure de bétaïne d'acide 3-(2-hydroxybenzoyloxy)-4-(triméthylammonio)-butyrique de formule (II) ou de la bétaïne d'acide 3-(2-hydroxybenzoyloxy)-4-(triméthylammonio)-butyrique de formule (III) audit acide arylsulfonique Ar-$SO_3H$ étant de 1:1 à 1:2.

**10.** Procédé selon l'une des revendications 6 à 9, comprenant en outre l'isolement de l'hydrosulfonate de bétaïne d'acide 3-(2-hydroxybenzoyloxy)-4-(triméthylammonio)-butyrique de formule (I) par cristallisation.

**11.** Procédé selon la revendication 10, ledit isolement étant effectué entre 0 et 40 °C.

**12.** Procédé selon l'une quelconque des revendications 6 à 11, ladite réaction d'un hydrohalogénure de bétaïne d'acide 3-(2-hydroxybenzoyloxy)-4-(triméthylammonio)-butyrique de formule (II) ou d'une bétaïne d'acide de 3-(2-hydroxybenzoyloxy)-4-(triméthylammonio)-butyrique de formule (III) avec un acide arylsulfonique Ar-$SO_3H$ comprenant :

(a) fournir une solution dudit hydrohalogénure de bétaïne d'acide 3-(2-hydroxybenzoyloxy)-4-(triméthylammonio)-butyrique de formule (II) ou de la bétaïne d'acide 3-(2-hydroxybenzoyloxy)-4-(triméthylammonio)-butyrique de formule (III) dans de l'eau ;
(b) ajouter ledit acide arylsulfonique Ar-$SO_3H$ ;
(c) chauffer le mélange réactionnel à une température supérieure à la température ambiante ; et
(d) isoler l'hydrosulfonate de bétaïne d'acide 3-(2-hydroxybenzoyloxy)-4-(triméthylammonio)-butyrique de formule (I) par cristallisation par refroidissement du mélange réactionnel à au moins la température ambiante,

le terme température ambiante se référant à une température de 15 à 30 °C.

**13.** Procédé selon l'une des revendications 6 et 8 à 12, ledit hydrohalogénure de formule (II) étant l'hydrochlorure de bétaïne d'acide 3-(2-hydroxybenzoyloxy)-4-(triméthylammonio)-butyrique.

**14.** Procédé selon l'une des revendications 6 et 8 à 13, ledit hydrosulfonate de bétaïne d'acide 3-(2-hydroxybenzoyloxy)-4-(triméthylammonio)-butyrique de formule (I) est un hydrobésilate de bétaïne d'acide (R)-(-)-3-(2-hydroxybenzoyloxy)-4-(triméthylammonio)-butyrique de formule (Ia), Y étant choisi parmi le bésylate et le tosylate et ledit acide arylsulfonique Ar-$SO_3H$ étant l'acide benzène-sulfonique ou l'acide para-toluène-sulfonique, et ledit hydrohalogénure de bétaïne d'acide 3-(2-hydroxybenzoyloxy)-4-(triméthylammonio)-butyrique de formule (IIa) étant un (R)-(-)-3-(2-hydroxybenzoyloxy)-4-(triméthylammonio)-butyrobétaïne de formule (II), $X^-$ étant choisi parmi le chlorure ($Cl^-$

), le bromure ($Br^-$) et l'iodure ($I^-$), et de préférence X étant le chlorure ($Cl^-$) ou le bromure ($Br^-$).

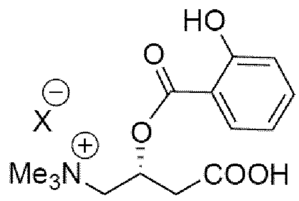

Formule (IIa)

**15.** Procédé selon l'une des revendications 7 à 13, ledit hydrosulfonate de bétaïne d'acide 3-(2-hydroxybenzoyloxy)-4-(triméthylammonio)-butyrique de formule (I) étant un hydrosulfonate de bétaïne d'acide (R)-(-)-3-(2-hydroxybenzoyloxy)-4-(triméthylammonio)-butyrique de formule (Ia), $Y^-$ étant choisi parmi le bésylate et le tosylate et ledit acide arylsulfonique Ar-$SO_3$H étant l'acide benzène-sulfonique ou l'acide para-toluène-sulfonique, et ladite bétaïne d'acide 3-(2-hydroxybenzoyloxy)-4-(triméthylammonio)-butyrique de formule (III) étant la bétaïne d'acide (R)-(-)-3-(2-hydroxybenzoyloxy)-4-(triméthylammonio)-butyrique de formule (IIIa).

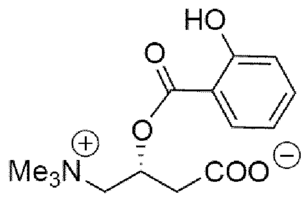

Formule (IIIa)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 553385 A
- EP 16186393 A